# EUROPEAN PATENT SPECIFICATION

(11) **EP 3 477 257 B1**
(45) Date of publication and mention of the grant of the patent: **19.05.2021**
(21) Application number: 17199355.3
(22) Date of filing: 31.10.2017
(51) Int. Cl.: G01B 21/02, G01B 21/10, A61B 5/107

(54) **AN ELECTRONIC DEVICE FOR OBTAINING A MEASURE OF A USER'S WRIST THICKNESS**
ELEKTRONISCHE VORRICHTUNG ZUM ERHALT EINES MASSES DER HANDGELENKDICKE EINES BENUTZERS
DISPOSITIF ÉLECTRONIQUE PERMETTANT D'OBTENIR UNE MESURE DE L'ÉPAISSEUR D'UN POIGNET D'UN UTILISATEUR

(43) Date of publication of application: 01.05.2019
(73) Proprietor: Vestel Elektronik Sanayi ve Ticaret A.S., 45030 Manisa (TR)
(72) Inventor: DÖNER, Çagdas, 45030 Manisa (TR)
(74) Representative: Flint, Adam

(56) References cited:
- WO-A1-2013/010276
- WO-A1-2014/207653
- US-A1- 2017 213 437

## Description

### Technical Field

The present disclosure relates to an electronic device for obtaining a measure of a user's wrist thickness.

### Background

Electronic devices, such as mobile phones, tablet computers, personal computers etc., may be used to monitor and track a number of values associated with a user's health, such as their weight, body fat percentage or body mass index (BMI). Typically, a user takes manual measurements of these values and enters the data manually into their electronic device. The electronic device can then be used to create and display graphs showing change in the values over time.

WO2013010276A1 discloses monitoring of water retention in a person by placing part of the body, such as an arm or ankle, between two capacitive electrodes and calculating the water content based on dielectric properties of the tissue. Another embodiment uses ultrasound to measure water content of tissue.

WO2014207653A1 discloses a wearable device for measuring a user's body dimensions which comprises a support structure, an extendable member, means for detecting the elongation of the extendable member and a connection interface.

US2017213437A1 discloses a monitoring device or monitoring health parameters of an individual. The monitoring device includes a stretchable component configured to fit securely around a body portion, and a sensor module coupled thereto. The sensor module is configured to obtain and transmit a circumference measurement of the body portion.

### Summary

According to an aspect disclosed herein, there is provide an electronic device for measuring a user's wrist thickness, the electronic device comprising: a strap for fitting round a user's wrist; a wrist thickness sensor housed in or on the strap, the wrist thickness sensor being constructed and arranged to measure a gap between the strap and the user's wrist when the strap encircles the user's wrist and to provide an output corresponding to the measured gap; and a controller configured to obtain a measure of the user's wrist thickness from the output of the wrist thickness sensor.

This allows for a convenient way of tracking a user's wrist thickness or change in the user's wrist thickness, which can be correlated with a change in the user's weight. This enables a user to monitor their weight whilst being unnecessary for the user to take time out of their day to weigh themselves and record their weight and/or without requiring access to a weighing machine or weighing scales. If the user has gained weight so that they have become overweight, or lost weight so that they have become underweight, the user can then take the appropriate corrective measures to return to a healthy weight.

In an example, the electronic device comprises a plurality of wrist thickness sensors.

In an example, the or at least one wrist thickness sensor comprises an ultrasonic sensor.

In an example, the or at least one wrist thickness sensor comprises a laser sensor.

In an example, the controller is configured to alert a user if an obtained measure of the user's wrist thickness has increased beyond a predetermined threshold value or decreased beyond a predetermined threshold value.

In an example, the controller is configured to alert a user if an obtained measure of the user's wrist thickness has increased beyond a predetermined threshold value or decreased beyond a predetermined threshold value over a predetermined threshold time period.

In an example, the electronic device comprises a display and wherein the controller is configured to alert the user by showing a message or image on the display

In an example, the electronic device comprises a transceiver and wherein the controller is configured to alert the user by using the transceiver to send a message to another electronic device.

In an example, the electronic device comprises a vibrator and wherein the controller is configured to alert the user by activating the vibrator.

In an example, the electronic device comprises a strap adjustment mechanism constructed and arranged to loosen and tighten the strap encircling the user's wrist as the user's wrist thickness increases or decreases.

### Brief Description of the Drawings

To assist understanding of the present disclosure and to show how embodiments may be put into effect, reference is made by way of example to the accompanying drawings in which:
Figure 1 shows schematically an axial cross-section view of an example of an electronic device; and
Figure 2 shows schematically a perspective view of the electronic device shown in Figure 1 engaged with a user's wrist.

### Detailed Description

Electronic devices, such as mobile phones, tablet computers, personal computers etc., may be used to monitor and track a number of values associated with a user's health, such as their weight, body fat percentage or body mass index (BMI). Typically, a user takes measurements of the values using some device or machine, such as weighing scales, and inputs the data manually into their electronic device. The electronic device can then be used to create and display graphs showing change in the values over time. A convenient way to display graphs showing a change in the various health related values over time to a user is to use wearable technology such as an activity tracker or a smartwatch. However, the known arrangements require the user to remember to for example weigh themselves from time to time, and also requires the user to have access to weighing scales or the like.

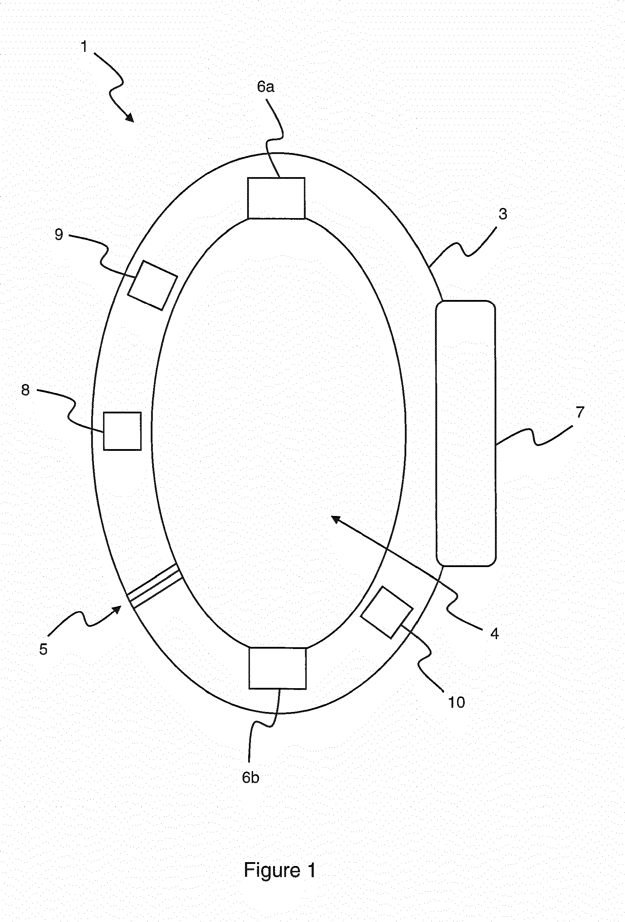

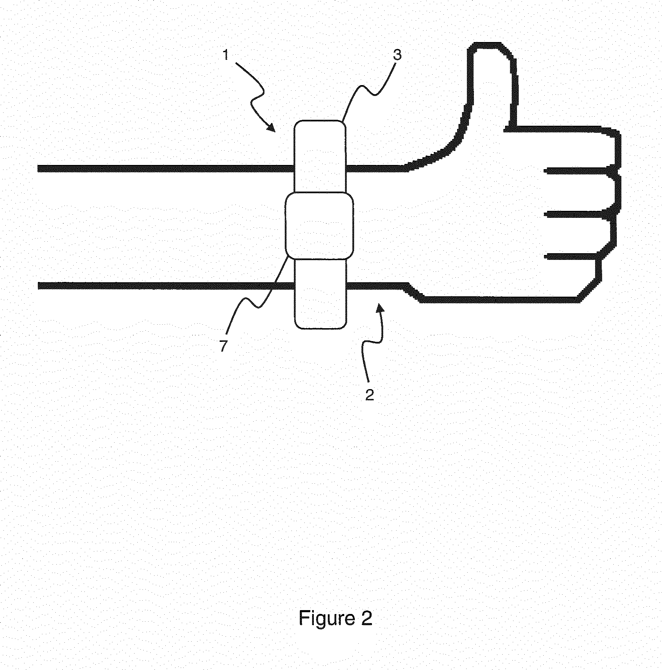

Referring now to Figure 1 and 2, there is shown schematically an example of an electronic device 1 for measuring the thickness of a user's wrist 2. In this example, the electronic device 1 is a smartwatch. In another example, the electronic device 1 may be another wearable technology device that is arranged to encircle or fit around a user's wrist 2, such as an activity tracker or a fitness tracker, etc.

The electronic device 1 includes a strap 3 that defines an aperture 4 therethrough. In this example, the strap 3 is generally annular. The strap 3 is sized such that the size of the aperture 4 is suitable for receiving and encircling a user's wrist 2. In this example, the strap 3 is quite rigid so that it sits in the same place on the user's wrist 2 each time it is fitted around the user's wrist 2. The strap 3 includes a pair of connectors 5, one provided on a first end and one provided on a second end. The connectors 5 can be unlocked from one another to allow for the aperture 4 to be opened so that a user can easily insert their wrist 2 into and through the aperture 4. The connectors 5 can also be locked to one another to encircle and secure the strap 3 to the user's wrist 2 once it has been inserted through the aperture 4. The connectors 5 may be any type of fastener that can cooperate to lock together, such as a pair of magnets, a buckle that engages with a hole in the strap 3, a clip, or a clasp.

The electronic device 1 also includes one or more wrist thickness sensors 6a, 6b for obtaining a measure of the thickness of the user's wrist 2. In this example, two wrist thickness sensors 6a, 6b are provided. In another example, a plurality of wrist thickness sensors 6a, 6b are provided. Each wrist thickness sensor 6a, 6b is housed within the strap 3. In another example, each wrist thickness sensor 6a, 6b is provided on the strap 3. The electronic device 1 may include a different number of wrist thickness sensors 6a, 6b. For example, having fewer wrist thickness sensors 6a, 6b may decrease the cost of manufacture, but having more wrist thickness sensors 6a, 6b may increase the accuracy of the measurement by the wrist thickness sensors 6a, 6b.

The wrist thickness sensors 6a, 6b may be a sensor that can sense a distance between itself and another object, such as a person's wrist or other body part. In this example, the wrist thickness sensors 6a, 6b are ultrasonic sensors. In another example, the wrist thickness sensors 6a, 6b may be laser sensors.

In this example, each wrist thickness sensor 6a, 6b is positioned and arranged so that it can obtain a measure of the distance between the sensor 6a, 6b and the user's wrist 2 when the electronic device is fitted to a user's wrist 2 (i.e. when the strap 3 has received and thus encircles the user's wrist 2). If the thickness of the user's wrist 2 increases then the distance between the respective sensors 6a, 6b and the user's wrist decreases. Similarly, if the thickness of the user's wrist 2 decreases then the distance between the respective sensors 6a, 6b and the user's wrist increases.

In order to obtain a measure of the distance between the wrist thickness sensors 6a, 6b and the user's wrist 2, each wrist thickness sensor 6a, 6b first emits a burst of sound towards the user's wrist 2. The burst of sounds travels through the air and contacts with the user's wrist 2. The burst of sounds then bounces off of the user's wrist 2 and is reflected back towards the wrist thickness sensor 6a, 6b from which it was originally emitted. The wrist thickness sensor 6a, 6b receives the reflected burst of sound and records the length of the time interval between the emission and reception of the burst of sound. This information can then be used to determine the distance between each wrist thickness sensor 6a, 6b and the user's wrist 2, as is explained below.

In another example, the wrist thickness sensors 6a, 6b are used to obtain a measure of the actual thickness of the user's wrist 2. To this end, each wrist thickness sensor 6a, 6b first obtains a measure of the distance between itself and the user's wrist 2. The wrist thickness sensors 6a, 6b then obtain a measure of the distance between themselves. This information can then be used to determine the actual thickness of the user's wrist 2, as is explained below. In another example, the distance between the wrist thickness sensors 6a, 6b is already known.

The electronic device 1 includes a display 7. The display 7 is an output device that, in normal use, produces an image display on its front face, which is visible to a user. In this example, the display 7 is an organic light-emitting diode (OLED) display screen. In other examples, the display 7 may be a liquid-crystal display (LCD) screen or some other type of display screen. In an example, the display 7 may include an input surface such as a touchscreen. The display 7 may be used to show information to a user such as the thickness of their wrist 2 over time. The display 7 may also be used to show an alert to a user if the thickness of their wrist 2 has increased or decreased beyond a threshold value.

The electronic device 1 includes a controller 8, which may be a processor or the like. The controller 8 is connected to the wrist thickness sensors 6a, 6b and the display 7. The controller 8 is configured to obtain a measure of the user's wrist thickness from the outputs of the wrist thickness sensors 6a, 6b. In particular, the controller 8 is configured to use the time interval information from the wrist thickness sensors 6a, 6b to calculate the distance between each wrist thickness sensor 6a, 6b and the user's wrist. This can be done by multiplying the time interval in seconds by the speed of sound in metres per second.

In another example, the controller 8 is configured to use the information from the wrist thickness sensors 6a, 6b to determine the actual thickness of the user's wrist 2. This can be done by subtracting the distance between each of the wrist thickness sensors 6a, 6b and the user's wrist 2 from the total distance between the wrist thickness sensors 2.

In an example, the controller 8 is configured so that it can detect if the strap 3 of the electronic device 1 was not encircling the user's wrist 2 when the controller 8 obtained a measure of the distance between each wrist thickness sensor 6a, 6b and the user's wrist 2. For example, if the controller 8 detects that there is a very large difference in a measurement made by a wrist thickness sensor 6a, 6b compared to a previous measurement (e.g. a change of 50% or more), the controller 8 determines that the electronic device 1 is either not mounted to the user's wrist 2 or is mounted incorrectly and therefore that the measurements are invalid. The controller 8 then discards the measurements. This ensures that only true measurements are obtained.

In this example, the electronic device 1 also includes a data storage 9. The data storage 9 stores information on the user's wrist thickness that is obtained by the wrist thickness sensors 6a, 6b.

It is known that the thickness of a person's wrist 2 may increase if the person gains weight and may decrease if a person loses weight. Therefore, if the controller 8 detects that the distance between the respective wrist thickness sensors 6a, 6b and the user's wrist 2 has decreased compared to a previous measurement then the thickness of the user's wrist 2 has increased since the previous measurement. The user may therefore have gained weight. Similarly, if the controller 8 detects that the distance between the respective wrist thickness sensors 6a, 6b and the user's wrist 2 has increased compared to a previous measurement then the thickness of the user's wrist 2 has decreased since the previous measurement. The user may therefore have lost weight. If the controller 8 detects that the distance between the wrist thickness sensors 6a, 6b and the user's wrist 2 has not changed then the thickness of the user's wrist 2 has not changed since the previous measurement and therefore the user's weight has remained the same.

In an example, the controller 8 is also configured to alert or inform a user when the thickness of their wrist 2 has increased by a threshold value, which may correspond with an increase in wrist thickness that indicates the user is overweight or has gained weight. The controller 8 may also be configured to alert or inform a user if the thickness of their wrist 2 has decreased by a predetermined threshold value, which may correspond with a decrease in wrist thickness that indicates the user is underweight or has lost weight.

The controller 8 may be configured to alert or inform a user if the thickness of their wrist 2 has increased or decreased by a predetermined threshold value or percentage over a predetermined threshold time period. For example, the controller 8 may be configured to alert a user if their wrist thickness has increased or decreased by more than, for example, 5% over a period of, for example, one month. This allows the controller 8 to inform a user if they are (probably) gaining or losing weight. Indeed, the controller 8 may be configured with multiple different combinations of predetermined threshold values or percentages and predetermined threshold time periods so as to monitor for a rapid or slow increase or decrease in the user's wrist thickness (and therefore their weight).

The controller 8 may obtain a measure of the thickness of the user's wrist 2 periodically from the wrist thickness sensors 6a, 6b. The controller 8 may then store this information in the data storage 9. In an example, the controller 8 obtains a measurement at regular intervals. For example, the controller 8 may obtain a measurements once per day or once per month. Additionally, or alternatively, the controller 8 may obtain a measure of the user's wrist thickness when the electronic device 1 is initially activated by user.

The controller 8 may be configured to alert a user by showing a message and/or and image on the display 7. In another example, the electronic device 1 may include a vibrator 10, which may be an eccentric rotating mass vibration motor or piezoelectric device, as is known in the art, and the controller 8 may be configured to alert a user by activating the vibrator 10. In an example, the controller 8 is configured to show a graph or chart of measurements of the user's wrist thickness over time on the display 7. The controller 8 may display the graph in response to user input. In an example, the electronic device 1 may include a transceiver (not shown) and the controller 8 may be configured to use the transceiver to send data on the user's wrist thickness to an external device such as another electronic device 1, for example a mobile phone or a tablet computer etc.

In some examples, the rigidity of the strap 3 is such that the strap 3 has the same fitting each time that it is secured around the user's wrist 2. This means that there is no (or very little) variation in the distance between the wrist thickness sensors 6a, 6b and the user's wrist 2 that is due to the fitting of the strap 3. This allows for repeatability of measurements of the gap between the strap 3 and the user's wrist 2 because any detected change in the distance is due to a change in the thickness of the user's wrist rather than due to incorrect fitting of the strap 3 to the user's wrist 2.

In an example, the electronic device 1 includes a strap adjustment mechanism. The strap adjustment mechanism is constructed and arranged so that it can automatically loosen and tighten the strap 3 around the user's wrist 2 if the user's wrist thickness increases or decreases (i.e. if the user gains or loses weight). This allows the strap 3 to stay securely fitted to the user's wrist 2 at all times so that it does not fall off. The strap adjustment mechanism may include a rolling mechanism that is controlled by a step motor. The motor may be connected to the controller 8 so that the controller 8 can adjust the length of the strap 3 to maintain a secure fit around the user's wrist 2 but also take the adjustment into account when calculating the measure of the user's wrist thickness. In another example, the strap adjustment mechanism is manually operable.

An example of use of the electronic device 1 shown in Figures 1 and 2 in monitoring a user's wrist thickness will now be described.

Initially, the electronic device 1 is not engaged with, or encircling, the user's wrist 2 (e.g. the user is not wearing the smartwatch).

The user, wanting to use the electronic device 1, opens the connectors 5 and puts their hand through the aperture 4 of the strap 3. The user then closes the connectors 5 so that the strap 3 encircles their wrist 2. The rigidity of the strap 3 means that every time it is secured around the user's wrist 2, it is fitted to and remains in the same position relative to the user's wrist 2. This is so that the distance between the wrist thickness sensors 6a, 6b and the user's wrist 2 does not change between measurements (unless of course the user's wrist thickness changes) so as to allow for repeat measurements of this distance to determine if the thickness of the user's wrist 2 has changed.

Once the strap 3 is encircled around their wrist 2, the user activates the electronic device 1 using a power switch (not shown) so that they may use the electronic device 1 in a normal manner. In this example, the wrist thickness sensors 6a, 6b then obtain the time interval between emission of a burst of sound to the user's wrist and reception of the burst of sound. The controller 8 then obtains a measure of the distance between each of the wrist thickness sensors 6a, 6b and the user's wrist by, in this example, multiplying the time interval in seconds by the speed of sounds in metres per second. The controller 8 stores this data in the data storage 9

At particular instances or time intervals the controller 8 again obtains measures of the distance between each of the wrist thickness sensors 6a, 6b and the user's wrist 2. For example, the controller 8 may take measurements as soon as the electronic device 1 is activated and after every 24 hours that it remains activated.

If the controller 8, based on the measurements, detects that a user's wrist thickness has increased or decreased by a predetermined threshold value over a predetermined time period then the controller 8 alerts to the user.

In this example, the predetermined threshold is a change of 5% in the user's wrist thickness and the predetermined time period is one month. Therefore, in this example, if the controller 8 detects that the thickness of the user's wrist has increased or decreased by more than 5% over a one month period then an alert is sent to the user so that they are informed of this change, which may be due to the user gaining or losing weight. In this example, the user is alerted by the controller 8 showing a warning message on the display 7 and activating the vibrator 10.

Alerting the user to the change in the thickness of their wrist 2 allows the user to take steps to mitigate the change in their weight by, for example, adjusting their diet and/or activity level.

An advantage of this arrangement is the provision of a convenient way of effectively tracking and monitoring a user's weight, or more specifically effectively tracking a change in the user's weight. It is therefore not necessary for a user to take time out of their day to weigh themselves and record their weight. In addition, this arrangement automatically alerts a user when it is probable that their weight has changed so that they are at an unhealthy weight. For example, if the user has gained weight so that they have become overweight, or lost weight so that they have become underweight. The user can then take the appropriate corrective measures to return to a healthy weight.

It will be understood that the processor or processing system or circuitry referred to herein may in practice be provided by a single chip or integrated circuit or plural chips or integrated circuits, optionally provided as a chipset, an application-specific integrated circuit (ASIC), field-programmable gate array (FPGA), digital signal processor (DSP), graphics processing units (GPUs), etc. The chip or chips may comprise circuitry (as well as possibly firmware) for embodying at least one or more of a data processor or processors, a digital signal processor or processors, baseband circuitry and radio frequency circuitry, which are configurable so as to operate in accordance with the exemplary embodiments. In this regard, the exemplary embodiments may be implemented at least in part by computer software stored in (non-transitory) memory and executable by the processor, or by hardware, or by a combination of tangibly stored software and hardware (and tangibly stored firmware).

Reference is made herein to data storage for storing data. This may be provided by a single device or by plural devices. Suitable devices include for example a hard disk and non-volatile semiconductor memory.

The examples described herein are to be understood as illustrative examples of embodiments of the invention. Further embodiments and examples are envisaged. Any feature described in relation to any one example or embodiment may be used alone or in combination with other features. In addition, any feature described in relation to any one example or embodiment may also be used in combination with one or more features of any other of the examples or embodiments, or any combination of any other of the examples or embodiments. Furthermore, equivalents and modifications not described herein may also be employed within the scope of the invention, which is defined in the claims.

## Claims

1. An electronic device (1) for obtaining a measure of a user's wrist thickness, the electronic device (1) comprising:
a strap (3) for fitting round a user's wrist;
a wrist thickness sensor (6a,6b) housed in or on the strap (3), the wrist thickness sensor (6a,6b) being constructed and arranged to measure a gap between the strap (3) and the user's wrist when the strap (3) encircles the user's wrist and to provide an output corresponding to the measured gap; and
a controller (8) configured to obtain a measure of the user's wrist thickness from said output of the wrist thickness sensor (6a,6b).

2. An electronic device (1) according to claim 1, comprising a plurality of wrist thickness sensors (6a,6b).

3. An electronic device (1) according to claim 1 or claim 2, wherein the or at least one wrist thickness sensor (6a,6b) comprises an ultrasonic sensor.

4. An electronic device (1) according to any of claims 1 to 3, wherein the or at least one wrist thickness sensor (6a,6b) comprises a laser sensor.

5. An electronic device (1) according to any of claims 1 to 4, wherein the controller (8) is configured to alert a user if an obtained measure of the user's wrist thickness has increased beyond a predetermined threshold value or decreased beyond a predetermined threshold value.

6. An electronic device (1) according to any of claims 1 to 5, wherein the controller (8) is configured to alert a user if an obtained measure of the user's wrist thickness has increased beyond a predetermined threshold value or decreased beyond a predetermined threshold value over a predetermined threshold time period.

7. An electronic device (1) according to claim 5 or claim 6, comprising a display (7) and wherein the controller (8) is configured to alert the user by showing a message or image on the display (7)

8. An electronic device (1) according to any of claims 5 to 7, comprising a transceiver and wherein the controller (8) is configured to alert the user by using the transceiver to send a message to another electronic device.

9. An electronic device (1) according to any of claims 5 to 8, comprising a vibrator and wherein the controller (8) is configured to alert the user by activating the vibrator.

10. An electronic device (1) according to any of claims 1 to 9, comprising a strap adjustment mechanism constructed and arranged to loosen and tighten the strap (3) encircling the user's wrist as the user's wrist thickness increases or decreases.

11. An electronic device (1) according to claim 10, wherein the strap adjustment mechanism is in communication with the controller (8) such that the controller (8), when obtaining a measure of the user's wrist thickness from the output of the wrist thickness sensor (6a,6b), can take into account the adjustment of the strap (3).

## Patentansprüche

1. Elektronische Vorrichtung (1) zum Erlangen eines Maßes einer Benutzerhandgelenkdicke, wobei die elektronische Vorrichtung (1) aufweist:
ein Band (3) zum Anbringen um ein Benutzerhandgelenk;
einen im oder am Band untergebrachten Handgelenkdickensensor (6a, 6b), wobei der Handgelenkdickensensor (6a, 6b) konstruiert und angeordnet ist, um einen Spalt zwischen dem Band (3) und dem Benutzerhandgelenk zu messen, wenn das Band (3) das Benutzerhandgelenk umringt, und um eine Ausgabe entsprechend dem gemessenen Spalt zu liefern; und
eine Steuerung (8), die konfiguriert ist, um aus der Ausgabe des Handgelenkdickensensors (6a, 6b) ein Maß der Benutzerhandgelenkdicke zu erlangen.

2. Elektronische Vorrichtung (1) nach Anspruch 1, aufweisend mehrere Handgelenkdickensensoren (6a, 6b).

3. Elektronische Vorrichtung (1) nach Anspruch 1 oder Anspruch 2, bei welcher der oder wenigstens ein Handgelenkdickensensor (6a, 6b) einen Ultraschallsensor aufweist.

4. Elektronische Vorrichtung (1) nach einem der Ansprüche 1 bis 3, bei welcher der oder wenigstens ein Handgelenkdickensensor (6a, 6b) einen Lasersensor aufweist.

5. Elektronische Vorrichtung (1) nach einem der Ansprüche 1 bis 4, bei welcher die Steuerung (8) konfiguriert ist, um einen Benutzer zu alarmieren, wenn ein erlangtes Maß der Benutzerhandgelenkdicke über einen vorbestimmten Schwellenwert gestiegen oder unter einen vorbestimmten Schwellenwert gesunken ist.

6. Elektronische Vorrichtung (1) nach einem der Ansprüche 1 bis 5, bei welcher die Steuerung (8) konfiguriert ist, um einen Benutzer zu alarmieren, wenn ein erlangtes Maß der Benutzerhandgelenkdicke über eine vorbestimmte Schwellenwertzeitdauer über einen vorbestimmten Schwellenwert gestiegen oder unter einen vorbestimmten Schwellenwert gesunken ist.

7. Elektronische Vorrichtung (1) nach Anspruch 5 oder Anspruch 6, aufweisend eine Anzeige (7) und bei welcher die Steuerung (8) konfiguriert ist, um den Benutzer durch Zeigen einer Mitteilung oder eines Bildes auf der Anzeige (7) zu alarmieren.

8. Elektronische Vorrichtung (1) nach einem der Ansprüche 5 bis 7, aufweisend einen Transceiver und bei welcher die Steuerung (8) konfiguriert ist, um den Benutzer durch Benutzen des Transceivers, um eine Mitteilung an eine andere elektronische Vorrichtung zu senden, zu alarmieren.

9. Elektronische Vorrichtung (1) nach einem der Ansprüche 5 bis 8, aufweisend einen Vibrator und bei welcher die Steuerung (8) konfiguriert ist, um den Benutzer durch Aktivieren des Vibrators zu alarmieren.

10. Elektronische Vorrichtung (1) nach einem der Ansprüche 1 bis 9, aufweisend einen Bandjustiermechanismus, der konstruiert und angeordnet ist, um das das Benutzerhandgelenk umringende Band (3) zu lockern und anzuziehen, wenn die Benutzerhandgelenkdicke zunimmt oder abnimmt.

11. Elektronische Vorrichtung (1) nach Anspruch 10, bei welcher der Bandjustiermechanismus mit der Steuerung (8) in Verbindung steht, sodass die Steuerung (8) beim Erlangen eines Maßes der Benutzerhandgelenkdicke aus der Ausgabe des Handgelenkdickensensors (6a, 6b) eine Justierung des Bandes (3) berücksichtigen kann.

## Revendications

1. Dispositif électronique (1) servant à obtenir une mesure d'épaisseur de poignet d'un utilisateur, le dispositif électronique (1) comprenant :
un bracelet (3) servant à s'ajuster autour du poignet d'un utilisateur ;
un capteur d'épaisseur de poignet (6a, 6b) qui est logé dans ou sur le bracelet (3), le capteur d'épaisseur de poignet (6a, 6b) étant construit et agencé de manière à mesurer un espacement entre le bracelet (3) et le poignet de l'utilisateur lorsque le bracelet (3) entoure le poignet de l'utilisateur, et de manière à obtenir une sortie correspondant à l'espacement mesuré ; et
un contrôleur (8) qui est configuré pour obtenir une mesure d'épaisseur de poignet de l'utilisateur à partir de ladite sortie du capteur d'épaisseur de poignet (6a, 6b).

2. Dispositif électronique (1) selon la revendication 1, comprenant une pluralité de capteurs d'épaisseur de poignet (6a, 6b).

3. Dispositif électronique (1) selon la revendication 1 ou 2, dans lequel le ou au moins un capteur d'épaisseur de poignet (6a, 6b) comprend un capteur à ultrasons.

4. Dispositif électronique (1) selon l'une quelconque des revendications 1 à 3, dans lequel le ou au moins un capteur d'épaisseur de poignet (6a, 6b) comprend un capteur laser.

5. Dispositif électronique (1) selon l'une quelconque des revendications 1 à 4, dans lequel le contrôleur (8) est configuré pour alerter un utilisateur si une mesure obtenue de l'épaisseur de poignet de l'utilisateur a augmenté au-delà d'une valeur de seuil prédéterminée ou a diminué au-delà d'une valeur de seuil prédéterminée.

6. Dispositif électronique (1) selon l'une quelconque des revendications 1 à 5, dans lequel le contrôleur (8) est configuré pour alerter un utilisateur si une mesure obtenue de l'épaisseur de poignet de l'utilisateur a augmenté au-delà d'une valeur de seuil prédéterminée ou a diminué au-delà d'une valeur de seuil prédéterminée sur une période de temps seuil prédéterminée.

7. Dispositif électronique (1) selon la revendication 5 ou 6, comprenant un affichage (7) et dans lequel le contrôleur (8) est configuré pour alerter l'utilisateur en affichant un message ou une image sur l'affichage (7).

8. Dispositif électronique (1) selon l'une quelconque des revendications 5 à 7, comprenant un émetteur-récepteur et dans lequel le contrôleur (8) est configuré pour alerter l'utilisateur en utilisant l'émetteur-récepteur pour envoyer un message à un autre dispositif électronique.

9. Dispositif électronique (1) selon l'une quelconque des revendications 5 à 8, comprenant un vibrateur et dans lequel le contrôleur (8) est configuré pour alerter l'utilisateur en activant le vibrateur.

10. Dispositif électronique (1) selon l'une quelconque des revendications 1 à 9, comprenant un mécanisme d'ajustement de bracelet qui est construit et agencé de manière à desserrer et serrer le bracelet (3) entourant le poignet de l'utilisateur lorsque l'épaisseur de poignet de l'utilisateur augmente ou diminue.

11. Dispositif électronique (1) selon la revendication 10, dans lequel le mécanisme d'ajustement de bracelet est en communication avec le contrôleur (8) de telle sorte que le contrôleur (8), lorsqu'il obtient une mesure de l'épaisseur de poignet de l'utilisateur à partir de la sortie du capteur d'épaisseur de poignet (6a, 6b), peut prendre en compte l'ajustement du bracelet (3).
